# EUROPÄISCHE PATENTSCHRIFT

(11) **EP 2 376 617 B1**
(45) Veröffentlichungstag und Bekanntmachung des Hinweises auf die Patenterteilung: **19.10.2016**
(21) Anmeldenummer: 09795350.9
(22) Anmeldetag: 17.12.2009
(51) Int. Cl.: C12M 1/107, B65D 90/08

(54) **BEHÄLTERWAND FÜR EINEN VON EINER FOLIE ABGEDECKTEN BEHÄLTER UND AUSSENSCHALUNG ZUR HERSTELLUNG DER BEHÄLTERWAND**
TANK WALL FOR A TANK COVERED BY A FILM AND OUTER SHELL FOR PRODUCING THE TANK WALL
PAROI DE CONTENANT POUR UN CONTENANT COUVERT D'UNE FEUILLE ET COFFRAGE EXTÉRIEUR POUR LA FABRICATION DE LA PAROI DE CONTENANT

(30) Priorität: 17.12.2008 DE 202008016776 U
(43) Veröffentlichungstag der Anmeldung: 19.10.2011
(73) Patentinhaber: MT-Energie Service GmbH, 27404 Zeven (DE)
(72) Erfinder: MARTENS, Christoph, 27404 Rockstedt (DE); BEHRENS, Jan, C.F., 27404 Seedorf (DE); MAACK, Peter, 21376 Salzhausen (DE)
(74) Vertreter: Hauck Patentanwaltspartnerschaft mbB
(86) Internationale Anmeldenummer: PCT/EP2009/009063
(87) Internationale Veröffentlichungsnummer: WO 2010/075981

(56) Entgegenhaltungen:
- EP-A2- 0 521 302
- DE-A1- 2 249 057
- DE-A1-102007 005 069
- DE-B- 1 064 869
- DE-B3-102006 035 227
- DE-U- 6 910 245
- DE-U1-202005 011 689

## Beschreibung

Die Erfindung bezieht sich auf eine Behälterwand nach Anspruch 1.

Behälter zum Lagern und Gären von Gärstoffen, beispielsweise in sogenannten Biogasanlagen, haben erhebliche Abmessungen. Sie sind zumeist siloartig ausgebildet und haben dementsprechend zumeist eine kreisrunde Grundfläche. Die Wand ist z.B. aus Beton gegossen, und die Abdichtung nach oben erfolgt oftmals mit Hilfe einer dachförmigen Folie, die durch Gasentwicklung oder durch Einfuhr von Druckluft in einem gespannten und angehobenen Zustand gehalten wird. Um die Folie am Rand wirksam an der Behälterwandung festzulegen, ist auch bekannt, im oberen Außenbereich der Wand ein Klemmprofil anzubringen, in welches der Folienrand hineingelegt und mit Hilfe eines Klemmschlauchs festgelegt wird.

Aus DE 10 64 869 A ist bekannt geworden, den Randbereich einer einen Behälter nach oben abdichtenden Folie in einen umlaufenden im Querschnitt U-förmigen nach oben offenen Schlitz einzulegen und anschließend einen aufblasbaren Schlauch einzulegen, um den Folienrand festzulegen. Aus DE 10 2006 035 227 B3 ist bekannt geworden, sowohl eine Gasspeicher- als auch Abdeckhaube aus Folienmaterial in einer C-förmigen außen am Behälter angebrachten Schiene festzulegen, wobei ein Klemmschlauch in die Schiene eingelegt wird zur Fixierung der Folien innerhalb der Schiene.

Je nach dem im Behälter herrschenden Gasdruck, Stützluftdruck oder den äußeren Einflüssen werden über die Folien dementsprechende Zugkräfte in die Schiene eingebracht. Es besteht daher die Gefahr, dass durch Verformung des Klemmprofils oder mangelnde Klemmkraft des Klemmschlauchs die Folie aus der Schiene herausrutscht.

Diesem kann in der Praxis durch festes Verflanschen der Folie(n) mit dem Behälter auch entgegengewirkt werden. Die Folie kann bis zum Zerreißen belastet werden. Nachteilig ist der erhöhte Aufwand beim Öffnen der Abdeckung, z.B. zu Wartungszwecken. Außerdem kann die Abdeckung beim Reißen der Folie ihre Funktion verlieren, was vermieden werden muss.

Je nach den Druckverhältnissen im Behälter und der an der Dachfolie angreifenden Windkraft werden erhebliche Zugkräfte auf den Folienrand ausgeübt. Daher besteht die Gefahr, dass der Folienrand herausgezogen wird, wenn die Zugkräfte einen bestimmten Wert übersteigen. Diese Gefahr wird erhöht, wenn der Winkel des Folienrandes zum zugeordneten Schenkel eines schienenförmigen Klemmprofils einen größeren Wert annimmt, so dass der Schenkel eine Verformung erleidet und nach oben gebogen und der Kraftschluss zwischen Folienrand und Klemmprofil reduziert wird.

Der Erfindung liegt die Aufgabe zugrunde, eine Behälterwand für einen Behälter mit einer Vorrichtung zur Festlegung einer den Behälter an der Oberseite dachförmig abschließenden Folie aus Kunststoff zu schaffen, die einfach herstellbar und montierbar ist und insbesondere auch hohen Zugkräften an der Folie widersteht. Diese Aufgabe wird durch die Merkmale des Anspruchs 1 gelöst..

Bei der Lösung der Erfindung ist dafür gesorgt, dass insbesondere bei höherer Zugbelastung der Folie die auf den zugewandten oberen Schenkel einwirkende Zugkraft im Wesentlichen als Druckkraft in den Schenkel eingeleitet wird und nur als kleineres Biegemoment auf den Schenkel wirkt. Es kommt daher nicht zu einer nachteiligen Verformung des Klemmprofils mit der Folge, dass der Folienrand herausgezogen wird.

Der Eintrittsschlitz des Klemmkanals liegt im Querschnitt gesehen in einer Ebene, die schräg nach unten gerichtet ist, d. h., er liegt im spitzen Winkel zur darüber liegenden Behälterwand. Erhöht sich im Behälter der Druck, führt dies zu einem Anheben der Dachfolie, wodurch der Winkel, vergrößert wird. Dadurch verringert sich die Auszugskraft am Klemmprofil. Durch ein Schrägstellen des Klemmprofils wird hingegen ein größerer Auszugswiderstand erreicht. Der Folienrand erstreckt sich z.B. in einem Winkel von 30° bis 45° zur Horizontalen. Der dem Folienrand zugekehrte Schenkel des Klemmprofils erstreckt sich vorzugsweise im gleichen Winkel. Somit ist bei der Erfindung auch bei höheren Auszugskraftwerten eine sichere Verankerung des Folienrands in dem Klemmprofil gewährleistet.

Nach einer Ausgestaltung der Erfindung ist der Klemmkanal bzw. das Klemmprofil von einer im Querschnitt U- oder vorzugsweise C-förmigen Profilschiene gebildet und vorzugsweise gekippt angeordnet. Die Behälterwand ist vorzugsweise aus Beton geformt und die Schiene kann in die Betonwand eingegossen sein.

Alternativ kann eine derartige Profilschiene außen an der Behälterwand befestigbar sein. Hierfür gibt es verschiedene Konstruktionsmöglichkeiten. Eine besteht nach einer Ausgestaltung der Erfindung darin, dass außen an die Behälterwand in Abständen vertikale Befestigungsprofile anbringbar sind, die vorzugsweise eine dem Querschnitt der Schiene angepasste Aussparung aufweisen, in welcher die Profilschiene gekippt eingesetzt, und vorzugsweise durch Verschweißung darin befestigt ist. Die Schiene, die vorzugsweise aus Längsabschnitten besteht, die anschließend miteinander verbunden werden, wird zunächst gebogen und anschließend mit den Befestigungsprofilen kraftschlüssig verbunden. Diese werden danach mit der Behälterwand verbunden, beispielsweise durch geeignete Anker. In einer weiteren Ausgestaltung der Erfindung ist in diesem Zusammenhang vorgesehen, dass das Befestigungsprofil im Querschnitt U-förmig ist, wobei die Aussparung in den Schenkeln geformt und der Steg des Befestigungsprofils mit der Behälterwand verbindbar ist.

Alternativ weist ein Haltemittel für die Profilschiene ein Winkelprofil auf, das z.B. mit der Schiene verschweißt ist, wobei das Winkelprofil mit der Außenseite der Behälterwand verbindbar ist.

Das Klemmprofil in Form einer im Querschnitt vorzugsweise C-förmigen Profilschiene kann in die Behälterwand eingegossen sein.
In einer weiteren Ausgestaltung der Erfindung ist die obere Kante oder der obere Schenkel der Profilschiene mit der Oberseite der Behälterwand bündig. Die Oberseite der Behälterwand kann nach einer weiteren Ausgestaltung der Erfindung zur Außenseite hin ansteigen.

Wie schon angedeutet ist die Erfindung besonders vorteilhaft, wenn die Profilschiene eine gekippte Position einnimmt, um die Auszugskraft zu erhöhen bzw. die Biegebelastung am zugeordneten Schenkel zu verringern. Bei außen an der Wand angebrachten Profilschiene wird vor allen Dingen der obere Schenkel stark mit der Zugkraft in der Folie belastet. Die Zugbelastung erzeugt ein Biegemoment. Die gekippte Anordnung ermöglicht einen Verlauf des der Folie zugekehrten Schenkels der Profilschiene, dass seine Erstreckungsrichtung annähernd parallel zu der Folie ist oder nur einen kleinen Winkel zu dieser einschließt. Dadurch kann eine geringer dimensionierte Profilschiene verwendet werden, um die gleichen Auszugskräfte wie bei einer herkömmlich angebrachten Profilschiene zu erreichen, deren Eintrittsschlitz z.B. in der Vertikalen liegt bzw. deren obere Schenkel einen größeren Winkel zur Folie bildet. Bei einer Einformung einer Profilschiene in das Material der Behälterwand braucht die Dicke des Materials der Profilschiene, die vorzugsweise aus Flachmaterial geformt ist, nur sehr gering sein, da die Kräfte weitgehend von dem umgebenden Material der Behälterwand aufgefangen werden. Auch bei der Anordnung einer Profilschiene in Aussparungen von vertikalen Befestigungsprofilen ist dafür gesorgt, dass einer Verbiegung des oberen Schenkels der Profilschiene ein erheblicher Widerstand entgegengesetzt ist, wodurch die Gefahr eines Herausziehens des Folienrandes stark reduziert ist.

Beton. Mit Hilfe einer Schalung kann auf einfache Weise in der Betonwand ein Klemmprofil angebracht werden. Eine Möglichkeit besteht erfindungsgemäß darin, dass eine im Querschnitt U- oder C-förmige Profilschiene an Außenschalungselementen lösbar anbringbar ist, wobei die Verbindung zwischen diesen Teilen von außen zugänglich ist. Beim Ausgießen der Schalung mit Beton wird die Profilschiene automatisch in die Wand eingebettet. Vor dem Entfernen der Schalung wird die Verbindung der übrigen Schalung mit der Profilschiene gelöst. Die Profilschiene kann auch in der Weise an einer Schalung angebracht sein, dass ihr oberer Schenkel der Erstreckungsrichtung der Folie angenähert ist bzw. ihr Eintrittsschlitz im Querschnitt in einer Ebene verläuft, die schräg nach unten weist.

Generell ist es erfindungsgemäß auch möglich, das Klemmprofil durch ein geeignetes Positivprofil an der Außenschalung zu bilden. Für das Positivprofil verwendeter Kunststoff ist geeignet, mit Hilfe von Wärme und/oder chemischer Substanzen entfernt werden, so dass in der Wandung ein Klemmkanal für die Befestigung des Folienrandes mit Hilfe eines geeigneten Klemmelements gebildet ist. Das Klemmelement ist entweder ein aufblasbarer Klemmschlauch oder das schon erwähnte Einlageprofil, um welches der Folienrand herumgelegt wird. Bei Auftreten einer Zugkraft wird der Folienrand zusammen mit dem Klemmelement im Bereich des Eintrittsschlitzes gegen die Innenseite des Klemmprofils bzw. seine Vorsprünge angelegt.

Nach einer Ausgestaltung der Erfindung weisen die Anbringmittel mindestens ein Loch an der Oberseite der Schalungselemente auf sowie eine Steck- oder Schraubverbindung für einen Profilhalter, der seinerseits so ausgebildet ist, dass er eine Profilschiene oder eine Schalungsschiene vor dem Gießen des Betons vorübergehend am Schalungselement nach dem Gießen des Betons durch Lösen der Anbringmittel und durch eine Kippbewegung mit der Profilschiene bzw. der Schalungsschiene außer Eingriff bringbar ist. Zu diesem Zweck kann der Profilhalter einen ersten Blechabschnitt aufweisen, der im annähernd rechten Winkel zum Eintrittsschlitz die einen Eintrittsschlitz für die Folie begrenzenden Vorsprünge der Profilschiene bzw. der Schalungsschiene von innen hintergreift und einen quer dazu angeordneten zweiten Blechabschnitt aufweist, der auf die Oberseite des Schalungselements auflegbar ist und mit dem Loch im Schalungselement ausrichtbares Befestigungsloch aufweist. Die Steckverbindung kann einen Stift aufweisen sowie einen Klemmabschnitt zur klemmenden Festlegung des zweiten Blechabschnitts an der Oberseite der Schalungselements durch Drehung von Stift und Klemmelement.

Schließlich kann unterhalb der Oberseite der Schalungselemente und unterhalb des Klemmprofils bzw. Schalungsprofils eine C-förmige Fahrschiene, die sich aus einzelnen Fahrschienenelementen zusammensetzt, mit dem Schalungselement verbunden werden, um die Fahrschiene in den Beton der Behälterwand einzubetten. Eine derartige Fahrschiene ermöglicht das Ansetzen eines Wagens an der Außenseite der Behälterwand, wobei er an Rollen, die in der Fahrschiene rollen, aufgehängt ist. Eine solche Konstruktion erübrigt unter Umständen die Anbringung eines um die Behälterwand herum erstreckten Laufsteges. Die Fahrschienenelemente können mit Profilschienenelementen von vorneherein verbunden sein, vorzugsweise durch Schweißung.

Ausführungsbeispiele der Erfindung werden nachfolgend anhand von Zeichnungen näher erläutert.
- Fig. 1a: zeigt schematisch die Draufsicht auf einen Behälter.
- Fig. 1b: zeigt einen Schnitt durch die Darstellung nach Fig. 1 entlang der Linie 2-2.
- Fig. 2: zeigt eine weitere Ausführungsform zur Festlegung einer Dachfolie.
- Fig. 3: zeigt eine Profilschiene an der Außenseite einer Behälter-wandung in gekippter Position.
- Fig. 4: zeigt ein Befestigungsprofil für eine Profilschiene nach Fig. 3.
- Fig. 5: zeigt angedeutet eine Außenschalung für eine Behälterwandung nach der Erfindung.
- Fig. 6: zeigt die Anbringung einer Profilschiene an der Schalung Fig. 5.
- Fig. 7: zeigt die Ansicht der Profilschiene nach Fig. 6 in Richtung Pfeil 13.
- Figuren 8 bis 11: zeigen verschiedene Ansichten eines Klemmbefestigungselements für einen Profilhalter nach Fig. 6.

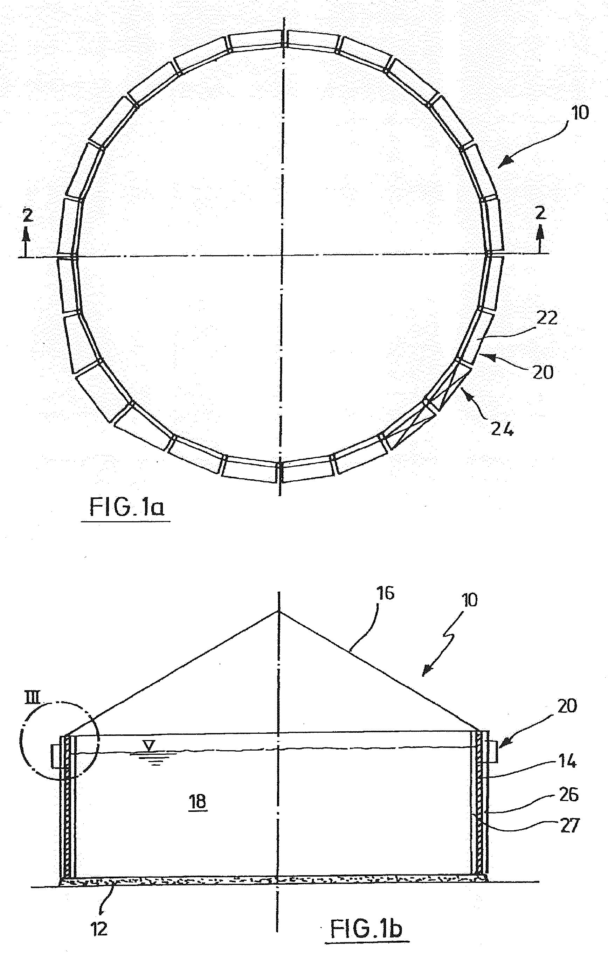

In den Fig. 1a und b ist ein Behälter 10 angedeutet, der eine Grundplatte 12 aus Beton aufweist und eine zylindrische Wand 14, ebenfalls aus Beton. Die Betonwand wird z. B. mit Hilfe einer Vielzahl von außen und innen angeordneten Schalungselementen hergestellt. Nach oben spannt sich mindestens eine Kunststofffolie 16 über den Behälter 10, wobei ihr Rand im oberen Bereich an der Außenseite der Wand 14 festgelegt wird, wie weiter unten noch zu beschreiben sein wird. Im Behälter befindet sich z. B. ein Gärstoff 18. An der Außenseite der Wand 14 ist ein umlaufender Laufsteg 20 mit Geländer gezeigt. Der Laufsteg besteht aus einzelnen Laufstegelementen, die auch miteinander verbunden sein können. Der Laufsteg ist für die Erfindung nicht von Bedeutung. In Fig. 1b ist außerdem eine Außenschalung 26 und eine Innenschalung 27 angedeutet. Sie dient, wie erwähnt, zur Herstellung der Wand, wobei die Schalung nach Errichtung der Betonwand 14 entfernt wird.

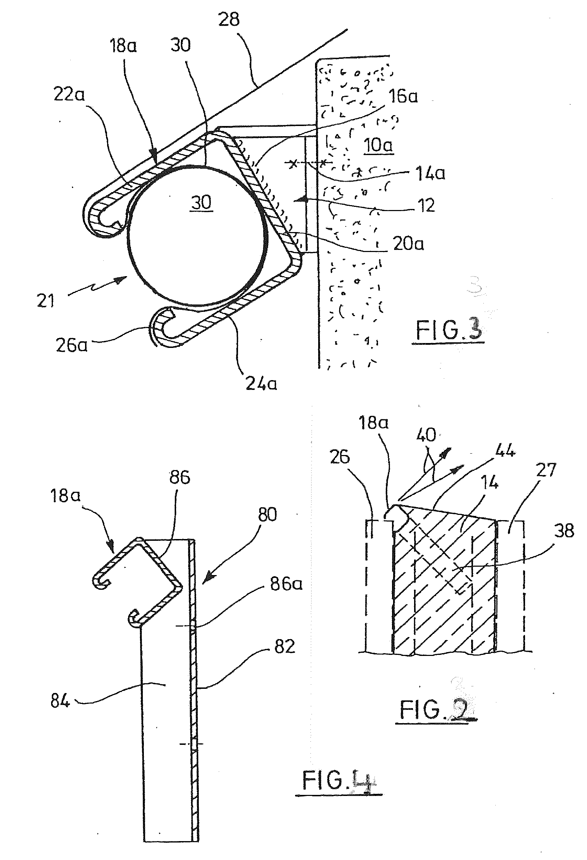

In Fig. 2 ist ein Schnitt durch den oberen Bereich der Behälterwandung 14 gezeigt. Man erkennt außerdem wiederum eine Außen- und Innenschalung 26, 27 nach Fig. 1b in Herstellung der Behälterwand aus Beton.

In Fig. 2 ist eine kastenförmige bzw. im Schnitt C-förmige Profilschiene 18a dargestellt, die beispielsweise eine Form hat, wie sie in Fig. 3 gezeigt ist. Hierauf wird weiter unten noch eingegangen. Bei der Herstellung wird die Profilschiene 18a mit der Außenschalung 26 lösbar verbunden (nicht gezeigt). Nach dem Formen der Wand 14 wird naturgemäß die Schalung 26, 27 entfernt, wobei zuvor die lösbare Verbindung zur Außenschale 26 getrennt wird. Die Profilschiene 18a ist dann mit der Wand 14 verbunden.

Die Profilschiene 18a besteht aus einzelnen Abschnitten bestimmter Länge, die vorzugsweise deutlich kleiner ist als die Umfangslänge des Behälters 10. Dabei kann ein Schienenabschnitt mit einem oder mehreren benachbarten Schalungselementen verbunden sein. Die Profilschiene 18a bzw. ihre einzelnen Abschnitte sind mit Ankerabschnitten versehen, die, wie bei 38 gezeigt, schräg nach unten in die Wand 14 hineinragen. Sie befinden sich bereits an der Profilschiene 18a, wenn sie an der Außenschalung 26 angebracht ist. In Fig. 2 ist mit den Pfeilen 40 die Zugrichtung einer Folie angedeutet, die in Fig. 1b mit 16 bezeichnet ist. Sie ist in der Profilschiene 18a festgelegt. Dies wird weiter unten noch beschrieben. Die unterschiedlichen Winkel der Zugrichtung 40 ergeben sich aus der Art der Folie (Gasspeicherfolie, Traglufthaube) und konstruktiven Gegebenheiten. Je nach Winkelgröße verändern sich Druck und Biegekraft am oberen Schenkel der Profilschiene.

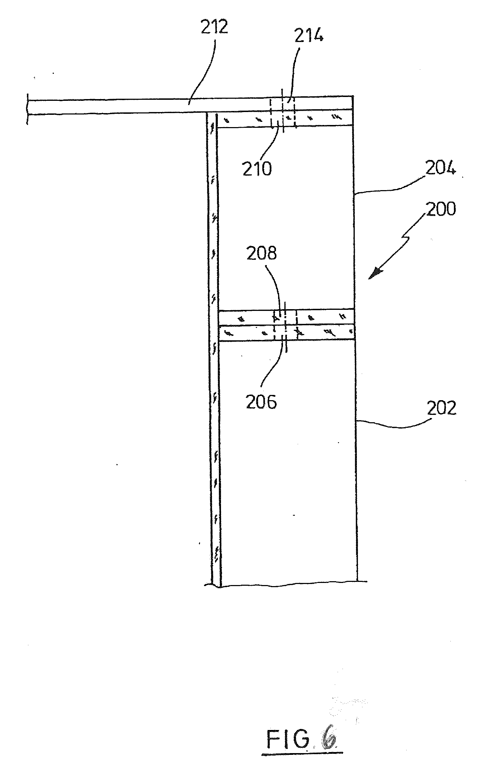

In Fig. 3 oder 6 ist die C-förmige Profilschiene dargestellt, wie sie in der Ausführungsform nach Fig. 2 in die Wand 14 eingebettet sein kann. Daher sollen Einzelheiten dieser Schiene später erläutert werden.

Die im Querschnitt C-förmige Profilschiene 18a nach Fig. 3 weist einen Steg 20a und zwei parallel beabstandete Schenkel 22a, 24a auf. Die Schenkel sind bei 26a umgebogen. Die gezeigte Profilschiene 18a ist leicht abgekippt mit schräg nach unten weisenden Schenkeln 22a, 24a. Ein Eintrittsschlitz 21 liegt somit in einer Ebene (im Querschnitt gesehen), die nach unten zeigt.

In Fig. 2 ist die Profilschiene 18a nach Fig. 3 so in die Wand 14 eingebettet, dass seine obere Kante mit der Oberseite der Wand 14 bündig ist. Diese Oberseite weist jedoch eine Steigung auf, wie bei 44 angedeutet. Die Steigung ist zur Außenseite hin gerichtet. Die Profilschiene 18a ist mit der Außenschalung 26 lösbar verbunden, wenn die Wand 14 hergestellt wird. Anschließend wird diese Verbindung gelöst, damit die Außenschalung 26 entfernt werden kann. Eine abgekippte Anbringung der Profilschiene 18a hat den Vorteil, dass deutlich größere Auszugswiderstände für den Folienrand erzielt werden bzw. eine günstige Beanspruchung der Profilschiene. Der obere Schenkel wird im wesentlichen in seiner Erstreckungsrichtung beansprucht und weniger auf Biegung. Der Folienrand ist mithin besonders sicher gehalten.

Die Klemmung einer Folie 28 in Fig. 3 findet mit Hilfe eines aufblasbaren Schlauches 30 statt, der in dem von der C-förmigen Profilschiene 18a gebildeten Klemmkanal eingelegt und anschließend aufgeblasen wird. Die Klemmung erfolgt vorzugsweise an der Innenseite von Steg 20a und Schenkeln 22a, 24a, wie ohne weiteres zu erkennen. Die nach innen gebogenen Abschnitte 26a verhindern, dass bei entsprechender Belastung der Schlauch 30 aus der Profilchiene 18a herausgezogen wird.

In Fig. 3 ist die Profilschiene 18a an der Außenseite einer Behälterwand 10a angebracht. An der Rückseite des Stegs 20a ist mit der Wand 10a ein Schenkel eines Winkelprofils 12 verschweißt, wie bei 16a angedeutet. Der andere Schenkel des Winkelprofils 12 ist, wie bei 14a angedeutet, in geeigneter Weise mit der Wand 10a verankert.

In Fig. 4 ist wiederum die Profilschiene 18a gemäß Fig. 3 dargestellt. Es soll dabei im Einzelnen nicht mehr beschrieben werden. In Fig. 3 ist ferner ein Befestigungsprofil 80 gezeigt. Es hat eine gewisse vertikale Länge, von z.B. 0,15 m. Es ist im Querschnitt U-förmig mit einem Steg 82 und zwei Schenkeln, von denen einer bei 84 gezeigt ist. Die Schenkel 84 sind im oberen Bereich des Befestigungsprofils 80 mit einer dreieckigen Aussparung 86 versehen. In diese wird die Profilschiene 18a in gekippter Stellung, beispielsweise mit einem Winkel von 45°, eingesetzt und darin befestigt. Die Befestigung wird jedoch erst vorgenommen, nachdem Abschnitte der Profilschiene 18a in die richtige Rundform gebogen werden, entsprechend dem Außenradius des Behälters, an dem die Profilschiene 18a angebracht werden soll. Im Steg des Befestigungsprofils 80 sind Löcher 86a geformt, so dass das Befestigungsprofil 80 mit Hilfe von Schrauben bzw. Ankern vertikal an die Außenwand eines Behälters angebracht werden kann, beispielsweise der Wand 10a in Fig. 3.

Statt ein Klemmprofil in Form einer Profilschiene in die Wand einzubetten, kann ein Positiv-Profil aus Kunststoff mit der Schalung verbunden werden, das anschließend aus der Wand entfernt wird, z. B. durch Wärme oder ein Reagens. Danach verbleibt in der Wand ein Klemmprofil mit der gleichen Funktion wie der einen Klemmschiene.

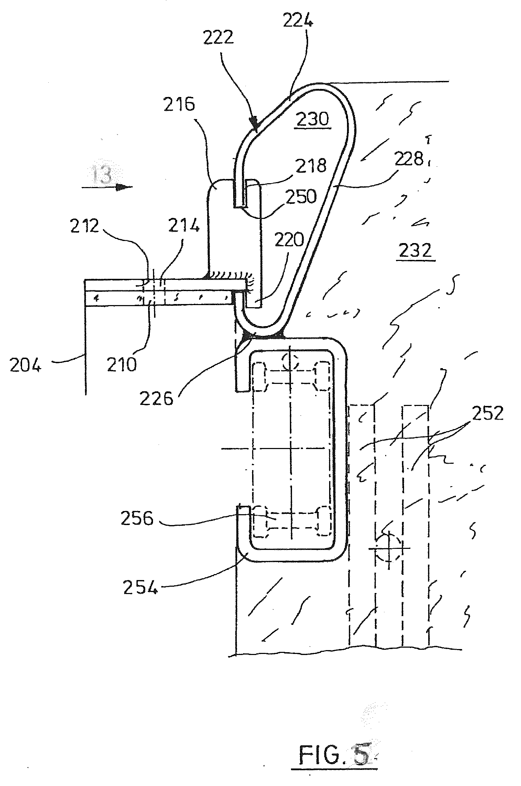

Fig. 5 zeigt angedeutet eine Schalung 200 für einen eine Betonwand aufweisenden Behälter, und zwar die Außenschalung. Die Außenschalung besteht aus unteren Schalungselementen 202, die ringförmig aufgestellt werden und dadurch die Außenwand des nicht gezeigten Behälters formen. Auf der Oberseite der unteren Schalungselemente 202 sind obere Schalungselemente 204 aufgesetzt und beispielsweise durch eine Schraubverbindung miteinander verbunden. Zu diesem Zweck weisen die Schalungselemente 202, 204 ausgerichtete Befestigungslöcher 206 bzw. 208 auf. Die oberen Befestigungselemente 204 formen den oberen Behälterrand aus. Sie haben an der Oberseite ebenfalls Löcher, von denen eines bei 210 dargestellt ist. In Fig. 5 ist außerdem ein Blechabschnitt 212 zu erkennen mit einem Befestigungsloch 214, so dass der Blechabschnitt 212 an der Oberseite der Schalungselemente 204 befestigt werden kann.

In Fig. 6 ist ein oberes äußeres Schalungselement 204 angedeutet und auch der Blechabschnitt 212 mit seinem Befestigungsloch 214. Man erkennt, dass mit dem horizontalen Blechabschnitt 212 ein vertikaler Blechabschnitt 216 verschweißt ist, der am oberen Ende einen rechteckigen Schlitz 218 und am unteren Ende einen Vorsprung 220 aufweist. In Fig. 6 ist außerdem im Querschnitt eine Profilschiene 222 angedeutet, die in Form eines leicht zusammengedrückten C-Profils ausgebildet ist mit einer schrägen oberen Wand 224 oder Schenkel, einer weitgehend gekrümmten unteren Wand 226 oder Schenkel und einem Boden 228. Die Abmessungen der oberen und unteren Wand sind derart, dass die Breite des Klemmkanals 230 innerhalb der Profilschiene 222 im oberen Bereich deutlich größer ist als im unteren Bereich. Zu diesem Zweck ist auch der Boden 228 schräg angeordnet. Ihre obere Wand 224 verläuft schräg nach oben. Dadurch wird der Vorteil erhalten, dass wie etwa in Fig. 3 die Profilschiene 222 den Zug an der nicht gezeigten Folie als Druckkraft im oberen Schenkel 224 aufnehmen kann. Der Klemmkanal 230 ist im Übrigen zur Aufnahme eines Flachprofils geeignet, um eine Folie im Klemmkanal 230 festzulegen.

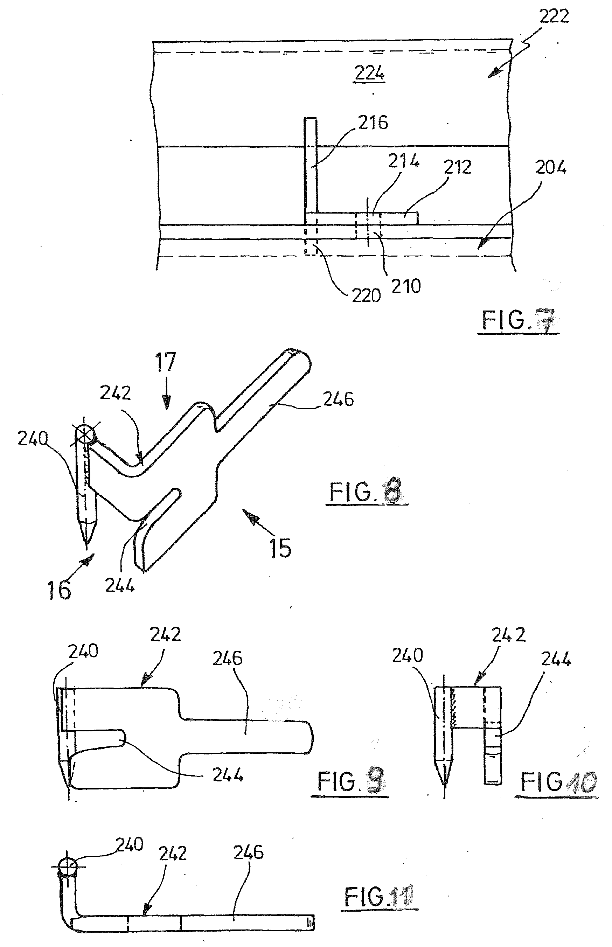

Die Blechabschnitte 212, 216 sind Teil eines Profilhalters, um die Profilschiene 222 vor dem Einbetten in die Wand 232 an dem Schalungselement 204 zu halten (Fig. 7). Mit Hilfe einer Vorrichtung, die in den Figuren 14 bis 17 dargestellt ist, kann der Profilhalter wirksam an dem Schalungselement 204 befestigt werden. Zu diesem Zweck weist die Vorrichtung nach den Figuren 8 bis 11 einen Stift 240 auf, der an einem Winkelabschnitt 242 aus Stahlblech befestigt ist, beispielsweise durch Schweißung. An dem dem Stift 240 entfernten Schenkel des Winkelabschnitts 242 ist ein Schlitz 244 geformt. Mit dem Winkelabschnitt 242 ist ein Griffabschnitt 246 verbunden. Bei der Befestigung des Blechabschnitts 212 am Blechabschnitt an der Oberseite des Schalungselements 204 wird der Stift durch die Löcher 214, 210 gesteckt. Dadurch ist der Schlitz 244 annährend zum Blechabschnitt 212 und dem Blech des Schalungselements 204 ausgerichtet. Durch eine entsprechende Drehung der Vorrichtung nach den Figuren 8 bis 11 wird der Schlitz über die zugeordneten Bleche geschoben, die dadurch gegeneinander geklemmt werden. Auf diese Weise ist der Profilhalter sicher am Schalungselement festgelegt, kann jedoch auf einfache Weise auch wieder entfernt werden.

Wie in Fig. 6 ferner zu erkennen, ist dort die Betonwand 232 bereits fertig erstellt, wobei die Innenschalungselemente nicht gezeigt sind. Die Betonwand weist Bewehrungen 252 auf. Außerdem ist zu erkennen, dass eine Fahrschiene 254 mit C-Profil mit der Profilschiene 222 an der Unterseite durch Schweißung befestigt ist. Sie wird daher ebenfalls vom Profilhalter gehalten, bis eine Aushärtung des Betons erfolgt. Die Fahrschiene 254 dient zur Aufnahme einer oder mehrerer Rollen 256 für einen nicht gezeigten Wagen, der an der Außenseite der Wand 232 aufgehängt ist und mit Hilfe der Fahrschiene 254 um den Behälter herum gefahren werden kann.

## Patentansprüche

1. Behälterwand, vorzugsweise aus Beton, insbesondere für Behälter zum Lagern und Gären von Gärstoffen, die nahe dem oberen Rand einen Klemmkanal zur Festlegung des Randes mindestens einer den Behälter an der Oberseite abdeckenden Folie aus Kunststoff aufweist, mit einem im oberen Bereich der Behälterwand angeordneten schräggestellten, ringförmigen Klemmprofil, das den inneren Klemmkanal mit einem nach außen offenen Eintrittsschlitz aufweist, wobei der Folienrand über den Eintrittsschlitz in den Klemmkanal einlegbar und mit Hilfe eines länglichen Klemmelements im Klemmkanal festlegbar ist, wobei der wandnahe Bereich der Folie einen Winkel zur Horizontalen einschließt und wobei das Klemmprofil einen oberen und einen unteren Schenkel hat, **dadurch gekennzeichnet, dass** der der Folie zugewandte obere Schenkel des Klemmprofils so geneigt ist, dass er sich bis zur maximalen zulässigen Zugbelastung der Folie annähernd parallel oder in einem kleinen Winkel zur Folie so erstreckt, dass eine von der Folie auf den oberen Schenkel aufgebrachte Kraft zu ihrem größeren Teil als Druckkraft auf diesen Schenkel wirkt.

2. Behälterwand nach Anspruch 1, **dadurch gekennzeichnet, dass** der Klemmkanal bzw. das Klemmprofil von einer im Querschnitt U- oder C- förmigen Profilschiene (18a, 36, 50, 222) gebildet und die Schiene vorzugsweise gekippt angeordnet ist, so dass ihr Eintrittsschlitz im Querschnitt der Schiene gesehen in einer schräg nach unten gerichteten Ebene angeordnet ist.

3. Behälterwand nach Anspruch 1 oder 2, **dadurch gekennzeichnet, dass** der obere Schenkel des Klemmprofils so geneigt ist, dass er sichparallel zur Folie erstreckt.

4. Behälterwand nach einem der Ansprüche 1 bis 3, **dadurch gekennzeichnet, dass** eine Profilschiene (36, 56, 222) zumindest teilweise in die Betonwand eingegossen ist.

5. Behälterwand nach Anspruch 1 oder 2, **dadurch gekennzeichnet, dass** die Profilschiene (18a, 222) an der Behälterwand von aussen befestigbar ist.

6. Behälterwand nach Anspruch 2, **dadurch gekennzeichnet, dass** der Winkel der Ebene des Eintrittsschlitzes des Klemmprofils gegenüber der sich darüber erstreckenden Behälterwand etwa 45° beträgt.

7. Behälterwand nach einem der Ansprüche 1 bis 6, **dadurch gekennzeichnet, dass** außen an die Behälterwand in Abständen vertikale Befestigungsprofile (80) anbringbar sind, die vorzugsweise eine dem Querschnitt der Profilschiene (18a, 36) angepasste Aussparung (86) aufweisen, in welche die Profilschiene (18a, 36) eingesetzt und an welchen die Profilschiene befestigbar ist.

8. Behälterwand nach Anspruch 7, **dadurch gekennzeichnet, dass** das Befestigungsprofil (80) im Querschnitt U-förmig ist, die Aussparung (86) in den Schenkeln vorgesehen ist, und der Steg (82) des Befestigungsprofils (80) mit der Behälterwand verbindbar ist, vorzugsweise mit Hilfe von Schraublöchern (86a) im Steg zur Aufnahme von Befestigungsschrauben.

9. Behälterwand nach Anspruch 7, **dadurch gekennzeichnet, dass** ein Haltemittel für die Profilschiene (18a, 222) ein Winkelprofil (12) aufweist, das mit der Schiene (18a, 222) kraftschlüssig verbunden ist, wobei das Winkelprofil mit der Behälterwand (10a) verbindbar ist.

10. Behälterwand nach Anspruch 4, **dadurch gekennzeichnet, dass** die obere Kante oder der obere Schenkel der Profilschiene mit der Oberseite der Behälterwand (14) bündig ist.

11. Behälterwand nach Anspruch 4, **dadurch gekennzeichnet, dass** die Profilschiene nahe der Oberseite der Behälterwand liegt und die Oberseite (44) der Behälterwand zur Aussenseite hin ansteigt.

12. Behälterwand nach Anspruch 1 oder 2, **dadurch gekennzeichnet, dass** die Profilschiene in Längsrichtung aus zwei oder mehreren Schienenabschnitten zusammengesetzt ist.

13. Behälterwand nach Anspruch 1, **dadurch gekennzeichnet, dass** das Klemmprofil (56) aus in der Längsebene geteilten Abschnitten besteht, die im Stegbereich kraftschlüssig miteinander verbunden sind.

14. Behälterwand nach einem der Ansprüche 1 bis 13, **dadurch gekennzeichnet, dass** der Klemmkanal (112) einen durch einen oberen und einen unteren Vorsprung (120, 122) gebildeten, verengten seitlichen Eintrittsschlitz (124) aufweist, dessen Höhe kleiner ist als die Höhe eines in den Klemmkanal einsetzbaren, länglichen Einlageprofils (126), um welche die Folie (130) herumlegbar ist, wobei die Querschnittsabmessungen von Kanal (112), Eintrittsschlitz (124) und Einlageprofil (126) derart sind, dass das Einlageprofil (126) in Schrägstellung zum Eintrittsschlitz über diesen in den Klemmkanal (112) einführbar und in annähernd paralleler Lage zu den Vorsprüngen gegen ein Herausbewegen aus dem Klemmkanal durch die Vorsprünge (110, 122) im Klemmkanal gehalten ist, wenn die um das Einlageprofil herumgelegte Folie (130) unter Spannung steht.

15. Behälterwand nach Anspruch 14, **dadurch gekennzeichnet, dass** das Einlageprofil (126) eine Sollbruchstelle (132) aufweist, wobei das Einlageprofil (126) im Wesentlichen um eine horizontale Achse knickt, wenn an der Folie (130) eine vorgegebene Kraft angreift.

## Claims

1. A container wall preferably made of concrete, in particular for containers for storing and fermenting fermented goods, which has a clamping channel close to the top edge for securing the edge of at least one film made of plastic that covers the top side of the container, having an oblique, angular clamping profile which is arranged in the top region of the container wall and has the inner clamping channel with an entry slot open to the outside, wherein the film edge can be inserted through the entry slot into the clamping channel and secured with the assistance of an elongated clamping element in the clamping channel, wherein the region of the film close to the wall encloses an angle with the horizontal, and wherein the clamping profile has a top and bottom leg, **characterized in that** the top leg of the clamping profile facing the film is angled such that it is approximately parallel or at a slight angle relative to the film up to the maximum permissible tension of the film such that the majority of a force exerted by the film on the top leg acts as pressure on this leg.

2. The container wall according to claim 1, **characterized in that** the clamping channel, or respectively the clamping profile, is formed by a profile rail (18a, 36, 50, 222) with a U-shaped or a C-shaped cross-section, and the rail is preferably arranged tilted such that its entry slot viewed in the cross-section of the rail is arranged in a plane which is inclined downwards.

3. The container wall according to claim 1 or 2, **characterized in that** the top leg of the clamping profile is angled such that it extends parallel to the film.

4. The container wall according to one of claims 1 to 3, characterized that a profile rail (36, 56, 222) is at least partially cast in the concrete wall.

5. The container wall according to claim 1 or 2, **characterized in that** the profile rail (18a, 222) can be attached to the container wall from the outside.

6. The container wall according to claim 2, **characterized in that** the angle of the plane of the entry slot of the clamping profile is about 45° relative to the container wall extending above it.

7. The container wall according to one of claims 1 to 6, **characterized in that** vertical attachment profiles (80) can be mounted on the outside of the container wall at intervals and preferably have a recess (86) that is adapted to the cross-section of the profile rail (18a, 36) into which the profile rail (18a, 36) is inserted, and to which the profile rail can be attached.

8. The container wall according to claim 7, **characterized in that** the attachment profile (80) has a U-shaped cross-section, the recess (86) is provided in the legs, and the bar (82) of the attachment profile (80) is connectable to the container wall, preferably with the assistance of screw holes (86a) in the bar for receiving fastening screws.

9. The container wall according to claim 7, **characterized in that** a retaining means for the profile rail (18a, 222) has an angle profile (12) that is connected to the rail (18a, 222) in a force fit, wherein the angle profile is connectable to the container wall (10a).

10. The container wall according to claim 4, **characterized in that** the top edge or top leg of the profile rail is flush with the top side of the container wall (14).

11. The container wall according to claim 4, **characterized in that** the profile rail is near the top side of the container wall, and the top side (44) of the container wall rises toward the outside.

12. The container wall according to claim 1 or 2, **characterized in that** the profile rail is composed of two or more rail sections in the longitudinal direction.

13. The container wall according to claim 1, **characterized in that** the clamping profile (56) consists of sections divided in the longitudinal plane which are connected to each other by a force fit in the web region.

14. The container wall according to one of claims 1 to 13, **characterized in that** the clamping channel (112) has a narrow, lateral entry slot (124) formed by a top and a bottom projection (120, 122), the height of the entry slot (124) being less than the height of an elongated insertion profile (126) which can be inserted into the clamping channel and around which the film (130) can be placed, wherein the cross-sectional dimensions of the channel (112), entry slot (124) and insertion profile (126) are such that the insertion profile (126) can, in an oblique position relative to the entry slot, be inserted through the entry slot into the clamping channel (112) and, in an approximately parallel position relative to the projections, be held from being removed from the clamping channel by projections (110, 122) in the clamping channel when the film (130) placed around the insertion profile is under tension.

15. The container wall according to claim 14, **characterized in that** the insertion profile (126) has a predetermined breaking point (132), wherein the insertion profile (126) bends substantially about a horizontal axis when a set force acts on the film (130).

## Revendications

1. Paroi de contenant, préférablement en béton, en particulier pour des contenants de stockage et de fermentation de substances fermentantes, qui comporte près du bord supérieur un canal de serrage pour la fixation du bord d'au moins une feuille en matière plastique recouvrant le contenant sur le côté supérieur, avec un profilé de serrage annulaire oblique agencé dans la partie supérieure de la paroi de contenant, qui comporte le canal de serrage interne avec une fente d'introduction ouverte vers l'extérieur, dans laquelle le bord de la feuille peut être introduit dans le canal de serrage via la fente d'introduction et fixé dans le canal de serrage à l'aide d'un élément de serrage longitudinal, dans laquelle la zone de la feuille proche de la paroi entoure une cornière à l'horizontale, et dans laquelle le profilé de serrage comporte une jambe supérieure et une jambe inférieure, **caractérisée en ce que** la jambe supérieure du profilé de serrage tournée vers la feuille est inclinée de telle sorte qu'elle s'étend jusqu'à la charge de traction maximale admissible de la feuille presque parallèlement à la feuille ou avec un faible angle par rapport à la feuille, de manière à ce qu'une force appliquée par la feuille sur la jambe supérieure agit principalement comme une force de poussée sur cette jambe.

2. Paroi de contenant selon la revendication 1, **caractérisée en ce que** le canal de serrage et le profilé de serrage sont constitués par un rail profilé à section transversale en forme de U ou de C (18a, 36, 50, 222), et **en ce que** le rail est préférablement agencé de manière inclinée de telle sorte que sa fente d'introduction vue en section transversale du rail est agencée dans un plan orienté obliquement vers le bas.

3. Paroi de contenant selon la revendication 1 ou 2, **caractérisée en ce que** la jambe supérieure du profilé de serrage est inclinée de telle sorte qu'elle s'étend parallèlement à la feuille.

4. Paroi de contenant selon l'une des revendications 1 à 3, **caractérisée en ce qu'**un rail profilé (36, 56, 222) est moulé au moins en partie dans la paroi en béton.

5. Paroi de contenant selon la revendication 1 ou 2, **caractérisée en ce que** le rail profilé (18a, 222) peut être fixé par l'extérieur à la paroi de contenant.

6. Paroi de contenant selon la revendication 2, **caractérisée en ce que** l'angle du plan de la fente d'introduction du profilé de serrage par rapport à la paroi de contenant qui s'étend en face est d'environ 45°.

7. Paroi de contenant selon l'une des revendications 1 à 6, **caractérisée en ce que** peuvent être installés de manière espacée depuis l'extérieur sur la paroi de contenant des profilés de fixation verticaux (80) qui présentent préférablement une encoche (86) adaptée à la section transversale du rail profilé (18a, 36), le rail profilé (18a, 36) pouvant être inséré dans ladite encoche et fixé à celle-ci.

8. Paroi de contenant selon la revendication 7, **caractérisée en ce que** le profilé de fixation (80) présente une section transversale en forme de U, **en ce que** l'encoche (86) est pourvue dans les jambes, et **en ce que** l'âme (82) du profilé de fixation (80) peut être connectée à la paroi de contenant, préférablement à l'aide de trous filetés (86a) dans l'âme pour recevoir des vis de fixation.

9. Paroi de contenant selon la revendication 7, **caractérisée en ce qu'**un moyen de retenue pour le rail profilé (18a, 222) comporte une cornière (12) qui est connectée au rail (18a, 222) par engagement de force, moyennant quoi la cornière peut être connectée à la paroi de contenant (10a).

10. Paroi de contenant selon la revendication 4, **caractérisée en ce que** le côté supérieur ou la jambe supérieure du rail profilé affleure avec le côté supérieur de la paroi de contenant (14).

11. Paroi de contenant selon la revendication 4, **caractérisée en ce que** le rail profilé se trouve près du côté supérieur de la paroi de contenant et le côté supérieur (44) de la paroi de contenant s'élève vers le côté externe.

12. Paroi de contenant selon la revendication 1 ou 2, **caractérisée en ce que** le rail profilé est assemblé en direction longitudinale à partir de deux sections de rail ou plus.

13. Paroi de contenant selon la revendication 1, **caractérisée en ce que** le profilé de serrage (56) est constitué de sections séparées dans le plan longitudinal qui sont connectées entre elles par engagement de force dans la zone de l'âme.

14. Paroi de contenant selon l'une des revendications 1 à 13, **caractérisée en ce que** le canal de serrage (112) comporte une fente d'introduction (124) latérale rétrécie, constituée par une projection supérieure et une projection inférieure (120, 122), dont la hauteur est inférieure à la hauteur d'un profilé d'insertion (126) longitudinal insérable dans le canal de serrage, autour duquel la feuille (130) peut être enroulée, dans laquelle les dimensions de la section transversale du canal (112), de la fente d'introduction (124) et du profilé d'insertion (126) sont telles que le profilé d'insertion (126) peut être introduit dans le canal de serrage (112) en position oblique par rapport à la fente d'introduction et par-dessus celle-ci, et est maintenu dans le canal de serrage en position pratiquement parallèle aux projections de manière à éviter une sortie hors du canal de serrage grâce aux projections (110, 122) quand la feuille (130) enroulée autour du profilé d'insertion se trouve sous tension.

15. Paroi de contenant selon la revendication 14, **caractérisée en ce que** le profilé d'insertion (126) comporte un point de rupture prédéterminé (132), moyennant quoi le profilé d'insertion (126) plie essentiellement autour d'un axe horizontal quand une force prédéterminée est appliquée sur la feuille (130).
